(19) Europäisches Patentamt
European Patent Office
Office européen des brevets

(11) **EP 3 618 080 B1**

(12) **EUROPEAN PATENT SPECIFICATION**

(45) Date of publication and mention
of the grant of the patent:
**27.03.2024 Bulletin 2024/13**

(51) International Patent Classification (IPC):
**G16H 50/20** (2018.01)     **G16H 50/70** (2018.01)

(21) Application number: **19192086.7**

(52) Cooperative Patent Classification (CPC):
**G16H 50/20; G16H 50/70**

(22) Date of filing: **16.08.2019**

(54) **REINFORCEMENT LEARNING FOR MEDICAL SYSTEM**

VERSTÄRKUNGSLERNEN FÜR EIN MEDIZINISCHES SYSTEM

APPRENTISSAGE PAR RENFORCEMENT POUR SYSTÈME MÉDICAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.08.2018 US 201862719125 P
23.05.2019 US 201962851676 P**

(43) Date of publication of application:
**04.03.2020 Bulletin 2020/10**

(73) Proprietor: **HTC Corporation
Taoyuan City 330, (TW)**

(72) Inventors:
• **CHEN, Yang-En**
**330 Taoyuan City (TW)**
• **TANG, Kai-Fu**
**330 Taoyuan City (TW)**
• **PENG, Yu-Shao**
**330 Taoyuan City (TW)**
• **CHANG, Edward**
**330 Taoyuan City (TW)**

(74) Representative: **Schmidbauer, Andreas Konrad
Wagner & Geyer Partnerschaft mbB
Patent- und Rechtsanwälte
Gewürzmühlstrasse 5
80538 München (DE)**

(56) References cited:
**US-A1- 2008 183 454    US-A1- 2018 046 773**

## Description

## BACKGROUND

Field of Invention

The disclosure relates to a machine learning method. More particularly, the disclosure relates to a reinforcement learning method for a medical system.

Description of Related Art

Recently the concept of computer-aided medical system has emerged in order to facilitate self-diagnosis for patients. The computer aided medical system may request patients to provide some information, and then the computer aided medical system may provide a diagnosis or a recommendation of the potential diseases based on the interactions with those patients.

US 2018/046773 A1 discloses medical system includes an interaction interface and an analysis engine. The interaction interface is configured for receiving an initial symptom. The analysis engine is communicated with the interaction interface. The analysis engine includes a prediction module. The prediction module is configured for generating symptom inquiries to be displayed on the interaction interface according to a prediction model and the initial symptom. The interaction interface is configured for receiving responses corresponding to the symptom inquiries. The prediction module is configured to generate a result prediction according to the prediction model, the initial symptom and the responses.

## SUMMARY

According to the present invention a medical system is provided as set forth in claim 1.

Preferred embodiments of the present invention may be gathered from the dependent claims.

It is to be understood that both the foregoing general description and the following detailed description are demonstrated by examples, and are intended to provide further explanation of the invention as claimed.

## BRIEF DESCRIPTION OF THE DRAWINGS

Embodiments of the invention will now be described with reference to the attached drawings in which:

FIG. 1 is a schematic diagram illustrating a medical system according to some embodiments of the disclosure;

FIG. 2A is a flow chart illustrating a control method by which a neural network model is trained by the medical system of FIG. 1 according to some embodiments of the disclosure;

FIG. 2B is a flow chart illustrating more detail of the control method shown in FIG. 2A according to some embodiments of the disclosure;

FIG. 2C is a flow chart illustrating more detail of the control method shown in FIG. 2A according to some embodiments of the disclosure;

FIG. 3 is a schematic diagram illustrating one medical record in the training data TD according to some embodiments of the disclosure;

FIG. 4 is a schematic diagram illustrating a structure of the neural network model according to some embodiments of the disclosure;

FIG. 5A is a schematic diagram illustrating states and an action determined by the control method in the symptom inquiry stage according to some embodiments;

FIG. 5B is a schematic diagram illustrating states and an action determined by the control method in the symptom inquiry stage according to some embodiments;

FIG. 5C is a schematic diagram illustrating states and an action determined by the control method in the symptom inquiry stage according to some embodiments;

FIG. 5D is a schematic diagram illustrating states and an action determined by the control method in the medical test suggestion stage according to some embodiments;

FIG. 5E is a schematic diagram illustrating states and an action determined by the control method in the result prediction stage according to some embodiments;

FIG. 6A is a demonstrational example about probability values and complement probability values corresponding to each of the medical test actions;

FIG. 6B is a schematic diagram illustrating several combinations formed by the medical test actions; and

FIG. 7 is a schematic diagram illustrating the medical system after the training of the neural network model is done.

## DETAILED DESCRIPTION

Reference will now be made in detail to the present embodiments of the disclosure, examples of which are illustrated in the accompanying drawings.

Wherever possible, the same reference numbers are used in the drawings and the description to refer to the same or like parts.

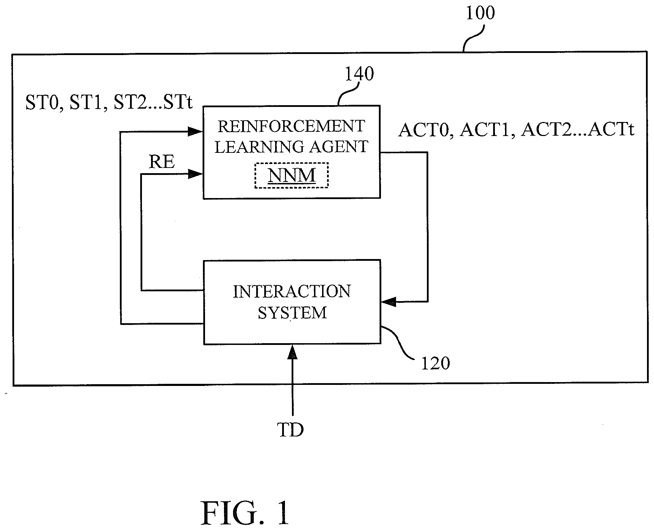

Reference is made to FIG. 1, which is a schematic diagram illustrating a medical system 100 according to some embodiments of the disclosure. As depicted in FIG. 1, the medical system 100 includes an interaction system 120 and a reinforcement learning agent 140. The interaction system 120 and the reinforcement learning agent 140 interact with each other, as described below, to train a neural network model NNM. In other words, the medical system 100 in FIG. 1 is in a training phase of training the neural network model NNM. The reinforcement learning agent 140 is configured to select sequential actions to cause the interaction system 120 to move from a current state to a next state, and subsequent states. The neural network model NNM is trained by the reinforcement learning agent 140 in reference to interactions between the interaction system 120 and the reinforcement learning agent 140 according to training data TD.

In some embodiments, the interaction system 120 and the reinforcement learning agent 140 can be implemented by a processor, a central processing unit or a computation unit. During a training phase of the medical system 100, the reinforcement learning agent 140 can be utilized to train the neural network model NNM (e.g., adjusting weights or parameters of nodes or interconnection links of the neural network model NNM) for selecting the sequential actions. During a training phase of the medical system 100, the interaction system 120 can be utilized as a supervisor of the training process on the reinforcement learning agent 140, such as the interaction system 120 will evaluate the sequential actions selected by the reinforcement learning agent 140 and provide corresponding rewards to the reinforcement learning agent 140. In some embodiments, the reinforcement learning agent 140 trains the neural network model NNM in order to maximize the rewards collected from the interaction system 120.

The neural network model NNM is utilized by the reinforcement learning agent 140 for selecting the sequential actions from a set of candidate actions. In some embodiments, the sequential actions selected by the reinforcement learning agent 140 include some symptom inquiry actions, one or more medical test actions (suitable for providing extra information for predicting or diagnosing the disease) and a result prediction action after the medical test actions and/or the symptom inquiry actions.

In some embodiments, the result prediction action includes a disease prediction action. In some other embodiments, the result prediction action includes a medical department recommendation action corresponding to the disease prediction action. In still other embodiments, the result prediction action include both of the disease prediction action and the corresponding medical department recommendation action. In following demonstrational embodiments, the result prediction action selected by the reinforcement learning agent 140 includes the disease prediction action. However, the disclosure is not limited thereto.

When the reinforcement learning agent 140 selects proper actions (e.g., some proper symptom inquiries, some proper medical test actions or a correct disease prediction action), corresponding rewards will be provided by the interaction system 120 to the reinforcement learning agent 140. In some embodiments, the reinforcement learning agent 140 trains the neural network model NNM to maximize cumulative rewards collected by the reinforcement learning agent 140 in response to the sequential actions. In some embodiments, the cumulative rewards can be calculated by a sum of a symptom abnormality reward, a test abnormality reward, a test cost penalty and a positive/negative prediction reward. Further details about how to calculate the cumulative rewards will be introduced in following paragraphs. In other words, the neural network model NNM will be trained to ask proper symptom inquiries, suggest proper medical tests and make the correct disease prediction at its best.

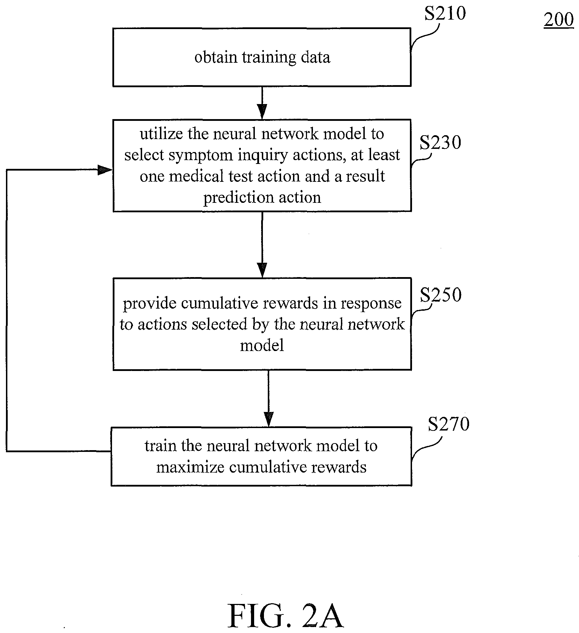

Reference is further made to FIG. 2A, which is a flow chart illustrating a control method 200 about how the neural network model NNM is trained by the medical system 100 in FIG. 1 according to some embodiments of the disclosure.

As shown in FIG. 1 and FIG. 2A, operation S210 of the control method 200 is performed by the interaction system 120 to obtain training data TD relating to the medical system 100. In some embodiments, the training data TD includes known medical records. The medical system 100 utilizes the known medical records in the training data TD to train the neural network model NNM. In an example, the training data TD can be obtained from data and statistics information from the Centers for Disease Control and Prevention (https://www.cdc.gov/datastatistics/index.html).

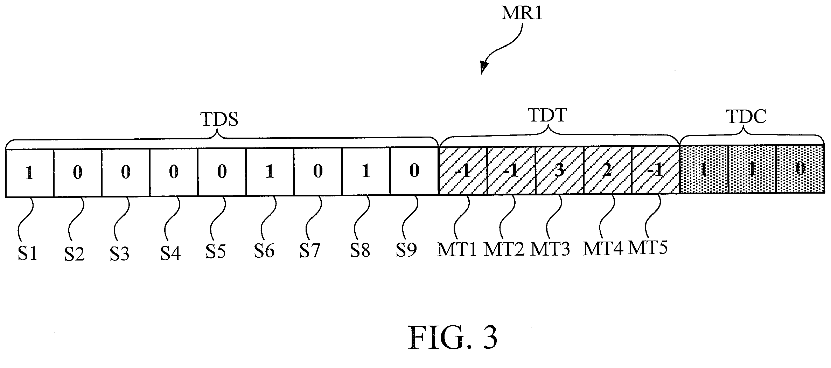

Reference is further made to FIG. 3, which is a schematic diagram illustrating one medical record MR1 in the training data TD according to some embodiments of the disclosure. In the embodiments shown in FIG. 3, the medical record MR1 in the training data TD relates to a diagnosed disease (not shown in figure) of a patient. The medical record MR1 includes diagnosed symptom information TDS, medical test information TDT and context information TDC. The diagnosed symptom information TDS in the medical record MR1 reveals symptoms, which occur to the patient with the diagnosed disease. The medical test information TDT in the medical record MR1 reveals results of medical tests performed on the patient in order to diagnose the diagnosed disease.

In some embodiments, the data bits "1" in the diagnosed symptom information TDS means that a patient mentioned in the medical record MR1 suffers the specific diagnosed symptom (e.g., cough, headache, chest pain, or dizzy). The data bits "0" in the diagnosed symptom information TDS means that the patient does not have the specific diagnosed symptom. As shown in FIG. 3, the diagnosed symptoms S1, S6 and S8 occurs to the patient, and the other symptoms S2-S5, S7 and S9 does not happen to the patient.

In some embodiments, the data bits "-1" in the medical test information TDT means that a specific medical test (e.g., blood pressure, chest x-ray examination, abdominal ultrasound examination, hemodialysis examination) has been performed to a patient mentioned in the medical record MR1, and the medical test result of the medical test is normal. The data bits "2" or "3" in the medical test information TDT mean that a specific medical test (e.g., blood pressure, chest x-ray examination, abdominal ultrasound examination or hemodialysis examination) has been performed to a patient mentioned in the medical record MR1, and also the medical test result of the medical test is abnormal, such as one index of the result is higher/lower than a standard range or an unusual shadow appears in the x-ray outcome. As the embodiment shown in FIG. 3, the medical test results of three medical tests MT1, MT2 and MT5 are normal, and the medical test results of two medical tests MT3 and MT4 are abnormal.

As shown in FIG. 3, the medical record MR1 indicates a relationship between the diagnosed disease, the diagnosed symptoms S1, S6 and S8 related to the diagnosed disease and the results of the medical tests MT1-MT5 performed for diagnosing the diagnosed disease. The medical record MR1 may record the diagnosed disease of a patient and also corresponding symptoms (the diagnosed symptoms S1, S6 and S8) occurring to the patient when the patient suffers the diagnosed disease. When a patient in another medical record (not shown) has another disease, the patient may have different symptoms corresponding to the disease. Even when two patients suffer the same disease, the two patients may have symptoms not exactly the same.

It is to be noticed that, the medical record MR1 having nine possible symptoms S1-S9 and five possible medical tests MT1-MT5 is illustrated in FIG. 3 for demonstration. However, the disclosure is not limited thereto. In some embodiments, the medical records in the training data TD may have about 200 to 500 possible symptoms and about 10 to 50 possible medical tests corresponding to about 200 to 500 possible diseases. The medical record MR1 merely illustrates a small part of the possible symptoms S1-S9 and the possible medical tests MT1-MT5 for briefly demonstrating.

The medical record MR1 in FIG. 3 shows that the patient has the diagnosed disease and the patient suffers the diagnosed symptoms S1, S6 and S8 (without the symptoms S2-S5, S7 and S9) and the medical test results of two medical tests MT3 and MT4 are abnormal (while the medical test results of three medical tests MT1, MT2 and MT5 are normal). In another medical record in the training data TD, when another patient suffering a different diagnosed disease may have different diagnosed symptoms and different medical test results, such that the data bits in this medical record will be different.

In some embodiments as illustrated in FIG. 3, the medical record MR1 may further include context information TDC of the patient. The context information TDC may indicate a gender, an age, a blood pressure, a mental status, a marriage status, a DNA table, or any other related information about the patient. In some embodiments, the context information TDC in the medical record MR1 is also utilized in training the neural network model NNM.

It is noticed that FIG. 3 illustrate one medical record MR1 in the training data TD for training the neural network model NNM. In practical applications, the training data TD may include about 100 to about 1000000 medical records. The training process discussed in operations S230-S270 will be repeated many times for each one of the medical records in the training data TD to optimize the trained neural network model NNM.

As shown FIG. 1 and FIG. 2A, operation S230 of the control method 200 is performed by the interaction system 120 and the reinforcement learning agent 140, to utilize the neural network model for selecting some symptom inquiry actions, at least one medical test action and a result prediction action.

As shown FIG. 1 and FIG. 2A, based on aforesaid actions (including the symptom inquiry actions, the at least one medical test action and the result prediction action) selected in operation S230, operation S250 of the control method 200 is performed by the interaction system 120. The operation S250 is performed by the interaction system 120 to provide corresponding cumulative rewards (a sum of a symptom abnormality reward, a test abnormality reward, a test cost penalty and a positive/negative prediction reward) to the reinforcement learning agent 140 based on aforesaid actions selected in operation S230.

As shown FIG. 1 and FIG. 2A, operation S270 of the control method 200 is performed by the reinforcement learning agent 140 to train the neural network model NNM in reference with the cumulative rewards, which are collected in response to the actions selected by the neural network model NNM. The neural network model NNM is trained to maximize the cumulative rewards, which are decided in reference with the test abnormality reward, the prediction reward and the test cost penalty.

When the operation S270 is finished, one training round relative to this medical record MR1 in the training data TD is completed. The control method 200 will return to operation S230 to start another training round relative to another medical record (not shown in figures) in the training data TD. After the neural network model NNM are trained with several medical records in the training data TD after several rounds, the neural network model NNM will be optimized in selecting the symptom inquiry actions, the medical test action(s) and the result prediction action.

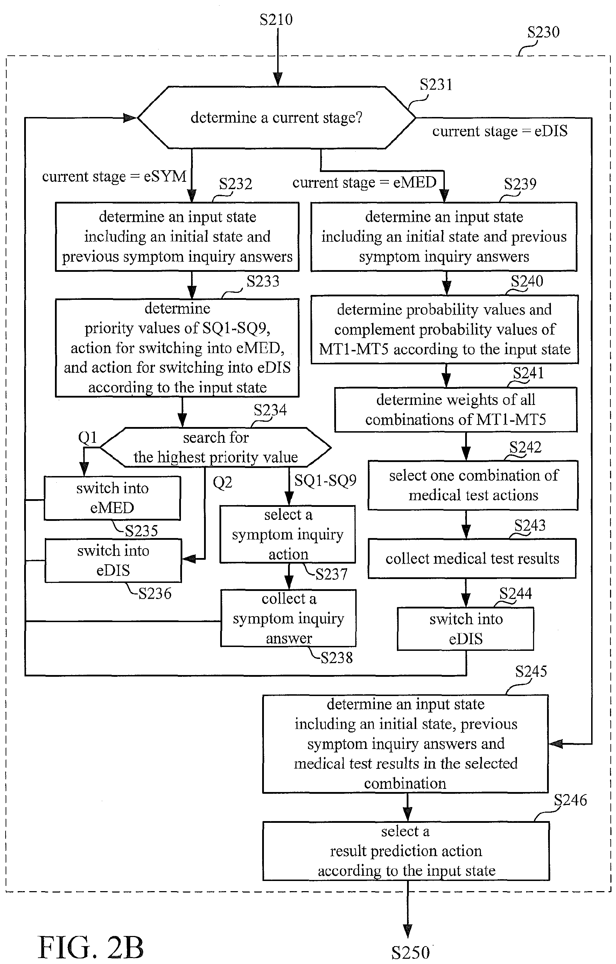

Reference is further made to FIG. 2B, which is a flow chart illustrating further operations S231-S246 in the operation S230 in FIG. 2A according to some embodiments of the disclosure.

As shown in FIG. 2B, the operations S231 is performed by the medical system 100 to determine a current stage of the control method 200 about how the neural network model NNM selects a current action. There are three different stages, which include a symptom inquiry stage eSYM, a medical test suggestion stage eMED and a result prediction stage eDIS in this embodiment. Initially, the control method 200 will enter the symptom inquiry stage eSYM. Later, the control method 200 may switch into the medical test suggestion stage eMED (in operation S235 from the symptom inquiry stage eSYM) or the result prediction stage eDIS (in operation S236 from the symptom inquiry stage eSYM or in operation S244 from the medical test suggestion stage eMED).

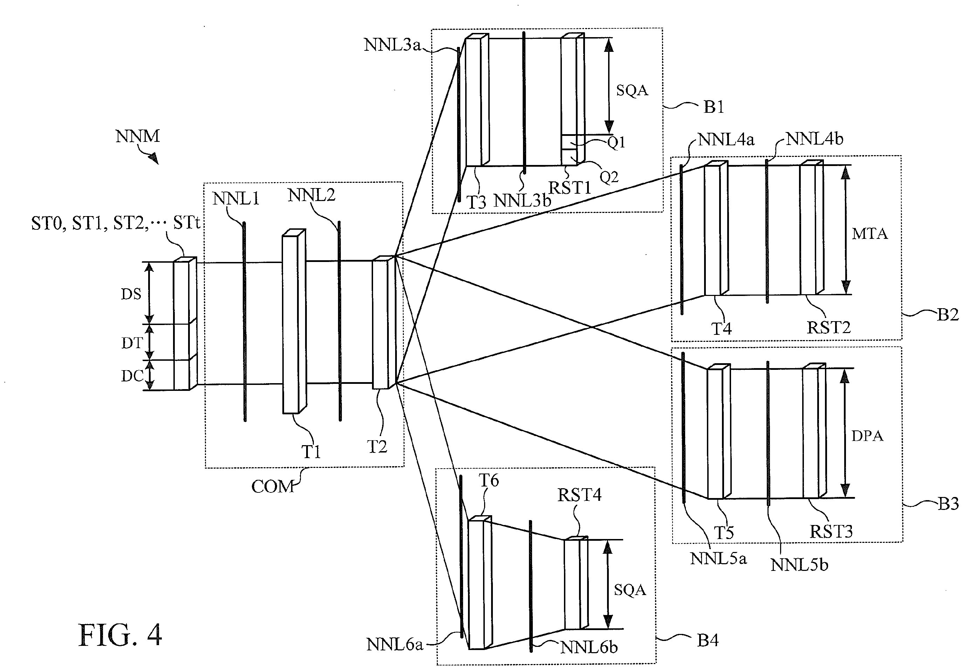

Reference is further made to FIG. 4, which is a schematic diagram illustrating a structure of the neural network model NNM according to some embodiments of the disclosure. As shown in FIG. 4, the neural network model NNM, utilized by the reinforcement learning agent 140, includes a common neural network portion COM, a first branch neural network portion B1, a second branch neural network portion B2, a third branch neural network portion B3 and a fourth branch neural network portion B4. The first branch neural network portion B1 is utilized to select the current action when the control method 200 in the symptom inquiry stage eSYM. The second branch neural network portion B2 is utilized to select the current action when the control method 200 in the medical test suggestion stage eMED. The third branch neural network portion B3 is utilized to select the current action when the control method 200 in the result prediction stage eDIS.

As shown in FIG. 4, the common neural network portion COM includes a neural network layer NNL1 to convert the input state ST0-STt into an intermediate tensor T1, and another neural network layer NNL2 to convert the intermediate tensor T1 into another intermediate tensor T2. In some embodiments, the neural network layer NNL1 and the neural network layer NNL2 can be fully-connection layers or convolution filter layers.

As shown in FIG. 4, the first branch neural network portion B1, the second branch neural network portion B2, the third branch neural network portion B3 and the fourth branch neural network portion B4 are respectively connected to the common neural network portion COM.

As shown in FIG. 4, the first branch neural network portion B1 includes a neural network layer NNL3a to convert the intermediate tensor T2 into another intermediate tensor T3, and another neural network layer NNL3b to convert the intermediate tensor T3 into the first result state RST1. In some embodiments, the neural network layer NNL3a can be a fully-connection layer or a convolution filter layer, and the neural network layer NNL3b can be a fully-connection layer, a convolution filter layer or an activation function layer. The first result state RST1 generated by the first branch neural network portion B1 is utilized to select one of a symptom inquiry action from the candidate inquiry actions SQA, an action for switching into the medical test suggestion stage eMED and another action for switching into the result prediction stage eDIS.

As shown in FIG. 4, the second branch neural network portion B2 includes a neural network layer NNL4a to convert the intermediate tensor T2 into another intermediate tensor T4, and another neural network layer NNL4b to convert the intermediate tensor T4 into the second result state RST2. In some embodiments, the neural network layer NNL4a can be a fully-connection layer or a convolution filter layer, and the neural network layer NNL4b can be a fully-connection layer, a convolution filter layer or an activation function layer. The second result state RST2 generated by the second branch neural network portion B2 is utilized to select a combination (including one or more medical test actions) of the medical test actions MTA.

As shown in FIG. 4, the third branch neural network portion B3 includes a neural network layer NNL5a to convert the intermediate tensor T2 into another intermediate tensor T5, and another neural network layer NNL5b to convert the intermediate tensor T5 into the third result state RST3. In some embodiments, the neural network layer NNL5a can be a fully-connection layer or a convolution filter layer, and the neural network layer NNL5b can be a fully-connection layer, a convolution filter layer or an activation function layer. The third result state RST3 generated by the third branch neural network portion B3 is utilized to select a result prediction action from the disease predictions DPA.

In some embodiments, the neural network layer NNL3b of the first branch neural network portion B1 and the neural network layer NNL5b of the third branch neural network portion B3 adopt the same activation function for generating the first result state RST1 and the third result state RST3. The neural network layer NNL4b of the second branch neural network portion B2 adopts another activation function (different from the neural network layer NNL3b/NNL5b) for generating the second result state RST2.

In the embodiments as shown in FIG. 4, the neural network layer NNL3b and the neural network layer NNL5b adopt a Softmax function, and the neural network layer NNL4b adopts a Sigmoid function. The Sigmoid function in the second branch neural network portion B2 allows the second branch neural network portion B2 to select multiple medical test actions simultaneously according to one input state.

It is noticed that, the Softmax function is usually utilized to select one action from candidate actions, and the Sigmoid function can be utilized to evaluate probabilities of several actions from candidate actions at the same time. In this embodiments, since the neural network model NNM has several branches (including the first branch neural network portion B1, the second branch neural network portion B2, the third branch neural network portion B3 and the fourth branch neural network portion B4), the second result state RST2 generated by the Sigmoid function can be utilized to select multiple medical test actions at the same time. On the other hand, the first result state RST1 can be utilized to select one symptom action in one round, and the third result state RST3 can be utilized to select one disease prediction in one round.

If the neural network model NNM does not include multiple branches, the neural network model NNM may have only one result state generated by the Softmax function, and the neural network model NNM cannot suggest multiple medical test actions at the same time based on the Softmax function. In this case, the neural network model will need to suggest one medical test, wait for an answer of the medical test, suggest another medical test and then wait for another answer.

As shown in FIG. 4, the fourth branch neural network portion B4 includes a neural network layer NNL6 to convert the intermediate tensor T2 into another intermediate tensor T6. In some embodiments, the neural network layer NNL6 can be a fully-connection layer or a convolution filter layer. The intermediate tensor T6 in the fourth branch neural network portion B4 is processed by another fully-connection layer into a fourth result state RST4 generated by the fourth branch neural network portion B4. The fourth result state RST4 generated by the fourth branch neural network portion B4 is utilized to reconstruct a possibility distribution of symptom features and medical test features.

Initially, when the control method 200 enters the symptom inquiry stage eSYM, operation S232 is performed by the interaction system 120 to determine an input state, which is transmitted to the reinforcement learning agent 140. The reinforcement learning agent 140 utilize the neural network model NNM to select an action according to the information carried in the input state.

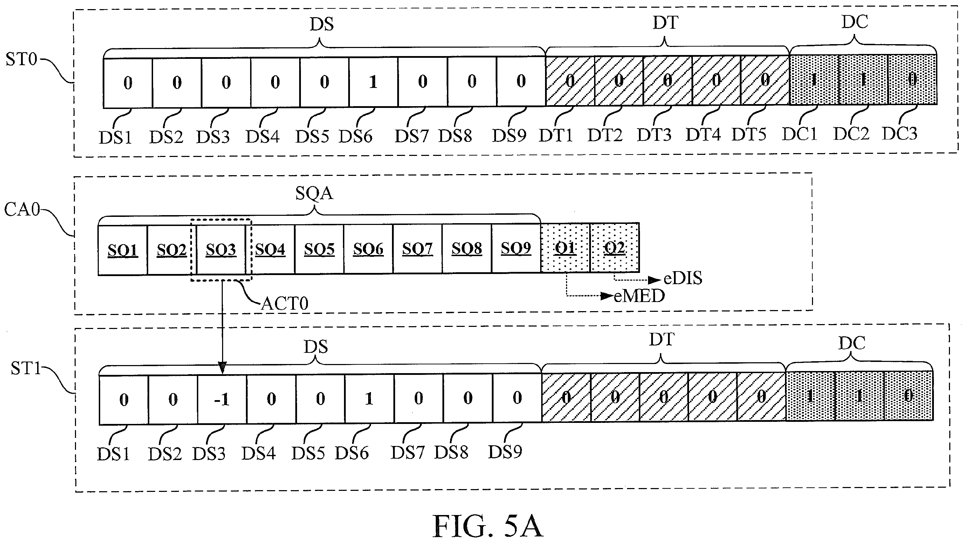

Reference is further made to FIG. 5A, which is a schematic diagram illustrating an input state ST0, an updated state ST1 and an action ACT0 determined by the control method 200 in the symptom inquiry stage eSYM according to some embodiments.

In an example, the interaction system 120 determines the input state ST0 as shown in embodiments of FIG. 5A. The state ST0 includes symptom data bits DS, medical test data bits DT and context data bits DC. Each data bit DS1-DS9 of the symptom data bits DS can be configured to 1 (a positive status means the symptom occurs), -1 (a negative status means the symptom does not occur) or 0 (an unconfirmed status means it is not sure whether the symptom occurs or not). Each data bit DT1-DT5 of the medical test data bits DT can be configured to -1 (means the medical test result is normal) or other number such as 1, 2 or 3 (means the medical test result is abnormal, over standard or below standard) or 0 (an unconfirmed status means it is not sure whether the medical test result is normal or abnormal). Each data bits DC1-DC3 of the context data bits DC indicate related information of the patient in the medical record. The data bits in the context data bits may indicate a gender, an age, a blood pressure, a mental status, a marriage status, a DNA table, or any other related information about the patient. For example, the data bit DC1 "1" can indicate the patient is a male, and the data bit DC3 "0" can indicate the patient is not married. In practical applications, the context data bits DC may include more data bits (not shown in figures) to record the age, the blood pressure, the mental status, the DNA table, or any other related information about the patient.

In embodiments as shown in FIG. 5A, the data bits DC1-DC3 of the context data bits DC can be duplicated from the context information TDC in the medical record MR1 as shown in FIG. 3.

In embodiments as shown in FIG. 5A, the data bit DS6 of the symptom data bits DS is set as "1" by the interaction system 120 according to the diagnosed symptom S6 in the medical record MR1 as shown in FIG. 3. In the initial state ST0, only the data bit DS6 is known, "1", and other data bits DS1-DS5 and DS7-DS9 of the symptom data bits DS are unconfirmed, "0".

As shown in FIG. 1, FIG. 2B and FIG. 5A, at the beginning, the operation S233 is performed, by the reinforcement learning agent 140 with the neural network model NNM, to determine priority values of all candidate actions CA0 in the symptom inquiry stage eSYM according to the input state ST0. In the embodiments shown in FIG. 5A, the reinforcement learning agent 140 with the neural network model NNM determines priority values of the symptom inquiry actions SQ1-SQ9, one stage switching action Q1 for switching from the symptom inquiry stage eSYM into the medical test suggestion stage eMED, and another stage switching action Q2 for switching from the symptom inquiry stage eSYM into the result prediction stage eDIS, according to the first result state RST1 generated by the first branch neural network portion B1 corresponding to the input state ST0.

As shown in FIG. 1, FIG. 2B and FIG. 5A, at the beginning, the operation S234 is performed, by the reinforcement learning agent 140, to search for the highest priority value from the priority values of the symptom inquiry actions SQ1-SQ9, and the stage switching actions Q1 and Q2. When the stage switching action Q1 has the highest priority value, operation S235 will be performed to switch into the medical test suggestion stage eMED. When the stage switching action Q2 has the highest priority value, operation S236 will be performed to switch into the result prediction stage eDIS.

As shown in FIG. 5A, the input state ST0 has not enough information to suggest a medical test or make a disease prediction. The priority values of the stage switching actions Q1 and Q2 determined in the first result state RST1 generated by the first branch neural network portion B1 of the neural network model NNM will be relatively low. In the embodiment of FIG. 5A, it is assumed that the priority value of the symptom inquiry action SQ3 is highest. Operation S237 is performed to select the symptom inquiry actions SQ3 by the reinforcement learning agent 140 with the neural network model NNM as a current action ACT0. When the symptom inquiry actions SQ3 is selected, a query about the third symptom (corresponding to the symptom S3 in FIG. 3) will be executed. Similarly, when different symptom inquiry actions SQA are selected, the query about the corresponding symptoms will be executed.

In some embodiments as shown in FIG. 1 and FIG. 2A, a budget "t" can be applied to the medical system 100 to decide how many symptom inquiries (i.e., how many actions from the symptom inquiry actions SQA) will be made before suggest a medical test (switching to the medical test suggestion stage eMED) or making a disease prediction (switching into the result prediction stage eDIS). In the following embodiments, the budget "t" is set at "3" for demonstration.

On the other hand, when the budget "t" is expired, the reinforcement learning agent 140 as shown in FIG. 1 and FIG. 2A will receive an expiration penalty, which will reduce the cumulative rewards collected by the reinforcement learning agent 140. The disclosure is not limited to that the budget "t=3". The budget "t" can be set at a positive integers larger than 1. In some embodiments, the budget "t" can be set about 5 to 9.

In some other embodiments, the budget "t" can be regarded as a maximum amount of symptom inquiries (i.e., how many actions from the symptom inquiry actions SQA) will be made before making the disease prediction (i.e., an action from the disease prediction actions DPA). However, the reinforcement learning agent 140 are not required to ask query a symptom for exact "t" times in every case in every cases (e.g., patients or medical records in the training data TD). If the reinforcement learning agent 140 already gathers enough information, the priority value of the stage switching action Q1 or Q2 will be highest to trigger the medical test suggestion stage eMED or the result prediction stage eDIS.

As shown in embodiments of FIG. 5A, in operation S237, the candidate action SQ3 of the symptom inquiry actions SQA is selected by the reinforcement learning agent 140 to be the action ACT0. In operation S238, the interaction system 120 will collect a symptom inquiry answer of the symptom inquiry actions SQ3. Based on the diagnosed symptoms in the medical record MR1 of the training data TD, the symptom inquiry answer of the symptom inquiry actions SQ3 will be set as "-1", which means the patient does not have the symptom S3.

An updated state ST1 (the updated state ST1 will be regard as an input state ST1 in the next round) is determined by the interaction system 120. As shown in FIG. 5A, in the updated state ST1, the data bit DS3 of the symptom data bits DS is changed from unconfirmed "0" into negative "-1", which means that the third symptom does not happen. The control method 200 will continue the operation S231 in reference with the updated state ST1 (as the new input state ST1).

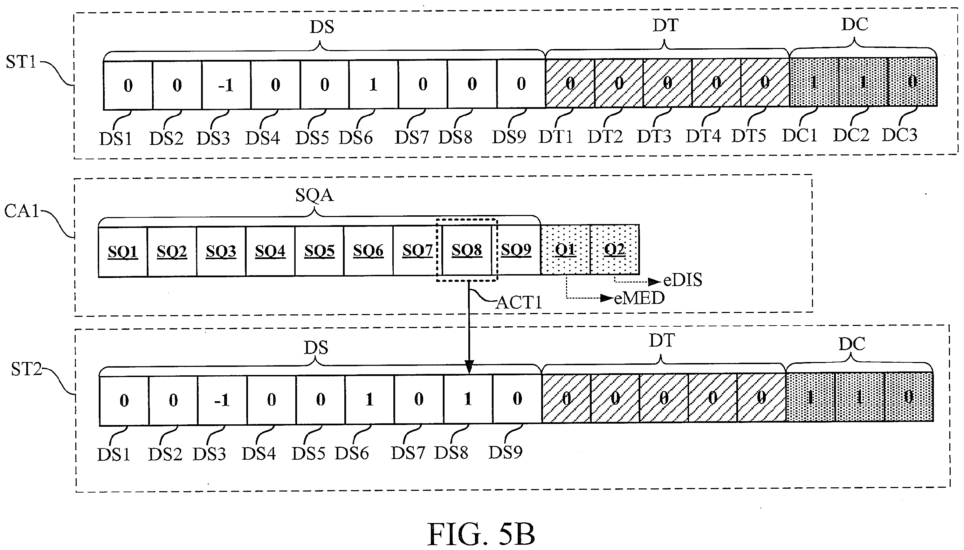

Reference is further made to FIG. 5B, which is a schematic diagram illustrating the input state ST1, an updated state- ST2 and another action ACT1 determined by the control method 200 in the symptom inquiry stage eSYM according to some embodiments.

As shown in FIG. 1, FIG. 2B and FIG. 5B, operation S231 is performed to determine a current stage, which is still in the symptom inquiry stage eSYM in this embodiment. Operation S232 is performed to determine the input state ST1, which include the initial state (e.g., DS6, and DC1-DC3) and the previous symptom inquiry answer (e.g., DS3). Operation S233 is performed to determine, by the reinforcement learning agent 140 with the neural network model NNM, to determine priority values of all candidate actions CA1 in the symptom inquiry stage eSYM according to the input state ST1. In the embodiments shown in FIG. 5B, the reinforcement learning agent 140 with the neural network model NNM determines priority values of the symptom inquiry actions SQ1-SQ9 and the stage switching actions Q1 and Q2, according to the first result state RST1 generated by the first branch neural network portion B1 corresponding to the input state ST1. Because the input state ST1 includes more information than the input state ST0, the priority values of the symptom inquiry actions SQ1-SQ9 and the stage switching actions Q1 and Q2 in this round shown in FIG. 5B will be determined to different levels from the last round shown in FIG. 5A. It is assumed that the symptom inquiry action SQ8 has the highest priority value.

In operation S237, the symptom inquiry action SQ8 is selected by the reinforcement learning agent 140 to be the action ACT1. In operation S238, the interaction system 120 will collect a symptom inquiry answer of the symptom inquiry actions SQ8. Based on the diagnosed symptoms in the medical record MR1 of the training data TD, the symptom inquiry answer of the symptom inquiry actions SQ8 will be set as "1", which means the patient have the symptom S8.

An updated state ST2 (the updated state ST2 will be regard as an input state ST2 in the next round) is determined by the interaction system 120. As shown in FIG. 5B, in the updated state ST2, the data bit DS8 of the symptom data bits DS is changed from unconfirmed "0" into "1", which means that the eighth symptom occurs on the patient. The control method 200 will continue the operation S231 in reference with the updated state ST2 (as a new input state ST2).

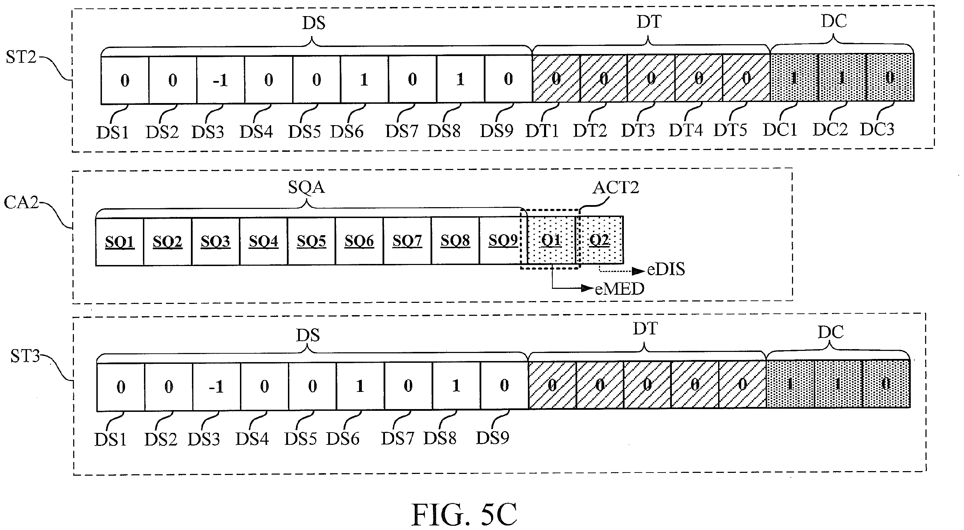

Reference is further made to FIG. 5C, which is a schematic diagram illustrating the input states ST2, an updated state ST3 and another action ACT2 determined by the control method 200 in the symptom inquiry stage eSYM according to some embodiments.

As shown in FIG. 1, FIG. 2B and FIG. 5C, operation S231 is performed to determine a current stage, which is still in the symptom inquiry stage eSYM in this embodiment. Operation S232 is performed to determine the input state ST2, which include the initial state (e.g., DS6, and DC1-DC3) and the previous symptom inquiry answers (e.g., DS3 and DS8). Operation S233 is performed to determine, by the reinforcement learning agent 140 with the neural network model NNM, to determine priority values (e.g., priority values) of all candidate actions CA2 in the symptom inquiry stage eSYM according to the input state ST2. In the embodiments shown in FIG. 5C, the reinforcement learning agent 140 with the neural network model NNM determines priority values of the symptom inquiry actions SQ1-SQ9 and the stage switching actions Q1 and Q2, according to the first result state RST1 generated by the first branch neural network portion B1 corresponding to the input state ST2. Because the input state ST2 includes more information than the input state ST1, the priority values of the symptom inquiry actions SQ1-SQ9 and the stage switching actions Q1 and Q2 in this round shown in FIG. 5C will be determined to different levels from the last round shown in FIG. 5B. It is assumed that the stage switching action Q1 has the highest priority value in this round. Operation S235 will be performed to switch into the medical test suggestion stage eMED and return to the operation S231. As shown in FIG. 5C, in this case, no symptom inquiry action is selected. Therefore, the updated state ST3 (the updated state ST3 will be regard as an input state ST3 in the next round) will be the same as the input state ST2. In this embodiment, the reinforcement learning agent 140 utilizes the neural network model NNM for selecting some symptom inquiry actions (e.g., SQ3 and SQ8) before the medical test action and the result prediction action. Therefore, the control method 200 will have enough information about what symptoms occur to the patient before suggesting a medical test or making a disease prediction.

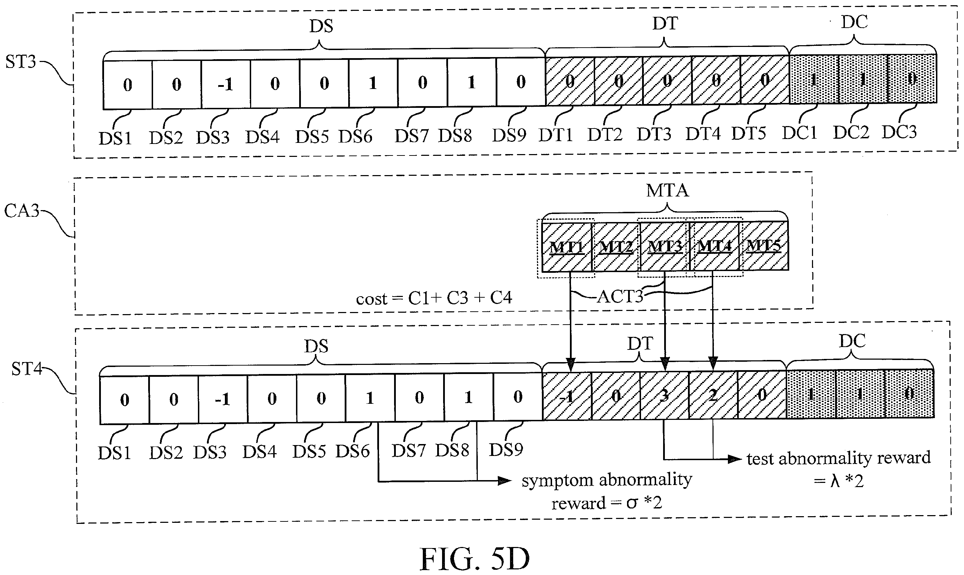

Reference is further made to FIG. 5D, which is a schematic diagram illustrating the input state ST3, an updated state ST4 and actions ACT3 determined by the control method 200 in the medical test suggestion stage eMED according to some embodiments.

As shown in FIG. 1, FIG. 2B and FIG. 5D, operation S231 is performed to determine a current stage, which is now in the medical test suggestion stage eMED in this embodiment.

Operation S239 is performed to determine the input state ST3, which include the initial state (e.g., DS6, and DC1-DC3) and the previous symptom inquiry answers (e.g., DS3 and DS8). Operation S240 is performed, by the reinforcement learning agent 140 with the neural network model NNM, to determine probability values and complement probability values of all candidate actions CA3 (which include five different medical test actions MT1-MT5) in the medical test suggestion stage eMED according to the state ST3.

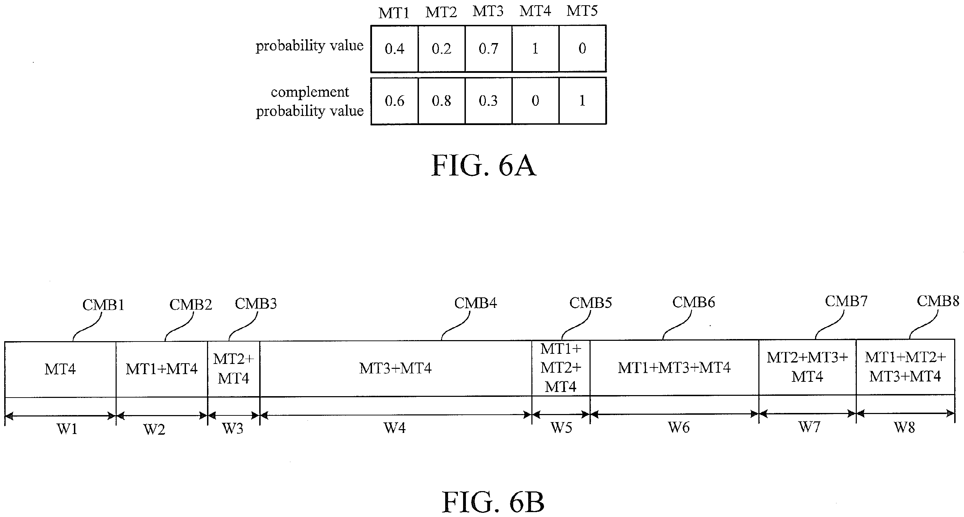

Reference is further made to FIG. 6A, which is a demonstrational example about the probability values and the complement probability values corresponding to each of the medical test actions MT1-MT5. In some embodiments, the probability values of the each of the medical test actions MT1-MT5 are generated in the second result state RST2, which is provided by the second branch neural network portion B2 adopting the second activation function (e.g., Sigmoid function). The probability values of the medical test actions MT1-MT5 will be values between 0 and 1. In this demonstrational example, each of the medical test actions MT1-MT5 has their probability value as 0.4, 0.2, 0.7, 1 and 0. The probability value values of the medical test actions MT1-MT5 stand for how important or necessary of the medical test actions MT1-MT5 to correctly predict the disease of the patient. The complement probability values are equal to "1 - probability value" of each of the medical test actions MT1-MT5. The complement probability values of the medical test actions MT1-MT5 are 0.6, 0.8, 0.3, 0 and 1. The medical test actions MT1-MT5 can be arranged into various combinations of medical test actions.

Reference is further made to FIG. 6B, which is a schematic diagram illustrating several combinations formed by the medical test actions MT1-MT5. As shown in FIG. 6B, the combination CMB1 includes performing the medical test action MT4 (without MT1, MT2, MT3 and MT5). The combination CMB2 includes performing the medical test actions MT1 and MT4 (without MT2-MT3 and MT5). The combination CMB3 includes performing the medical test actions MT2 and MT4 (without MT1, MT3 and MT5). The combination CMB4 includes performing the medical test actions MT3 and MT4 (without MT1, MT2 and MT5). The combination CMB5 includes performing the medical test actions MT1, MT2 and MT4 (without MT3 and MT5). The combination CMB6 includes performing the medical test actions MT1, MT3 and MT4 (without MT2 and MT5). The combination CMB7 includes performing the medical test actions MT2, MT3 and MT4 (without MT1 and MT5). The combination CMB8 includes performing the medical test actions MT1, MT2, MT3 and MT4 (without MT5).

Operation S241 is performed, by the reinforcement learning agent 140, to determine weights of all combinations of the candidate medical tests MT1-MT5 according to the probability values and the complement probability values.

The weight of one combination is a product between the probability values of selected tests and the complement probability values of non-selected tests. As shown in FIG. 6B, the weight W1 of the combination CMB1 can be calculate as a product of the probability value of MT4 and the complement probability values of MT1-MT3 and MT5. In other words, W1 = 0.6 * 0.8 * 0.3 * 1 * 1 = 0.144. As shown in FIG. 6B, the weight W2 of the combination CMB2 can be calculate as a product of the probability values of MT1 and MT4 and the complement probability values of MT2-MT3 and MT5. In other words, W2 = 0.4 * 0.8 * 0.3 * 1 * 1 = 0.096. As shown in FIG. 6B, the weight W3 of the combination CMB3 can be calculate as W3= 0.6 * 0.2 * 0.3 * 1 * 1 = 0.036. As shown in FIG. 6B, the weight W4 of the combination CMB4 can be calculate as W4 = 0.6 * 0.8 * 0.7 * 1 * 1 = 0.336. As shown in FIG. 6B, the weight W5 of the combination CMB5 can be calculate as W5 = 0.4 * 0.2 * 0.3 * 1 * 1 = 0.024. As shown in FIG. 6B, the weight W6 of the combination CMB6 can be calculate as W5 = 0.4 * 0.8 * 0.7 * 1 * 1 = 0.224. In a similar way, the weights W7 and W8 can be calculated.

In some embodiments, operation S242 is performed for randomly selecting one combination of medical test actions MT1-MT5 from the all combinations CMB1-CMB8 in reference with the weights W1-W8. In this case, one combination with the higher weight will have a higher chance to be selected. For example, the combination CMB4 and the combination CMB6 will have a higher chance to be selected compared to the combination CMB2 and the combination CMB3. In this embodiment shown in FIG. 5D, it is assumed that the combination CMB6 (with W6 = 0.224) is selected.

In some other embodiments, operation S242 is performed for selecting one combination of medical test actions MT1-MT5 from the all combinations CMB1-CMB8 with the highest one of the weights W1-W8.

Because the combination CMB5 (performing the medical test actions MT1, MT3 and MT4) are selected, the medical test actions MT1, MT3 and MT4 are selected as the current actions ACT3 simultaneously. Operation S243 is performed to collect medical test results corresponding to the medical test actions MT1, MT3 and MT4 according to the medical record MR1 in the training data TD. As shown in FIG. 5D, the data bit DT1 in the state ST4 of the medical test action MT1 is changed into "-1", which means a result of the medical test action MT1 is normal. The data bit DT3 in the state ST4 of the medical test action MT3 is changed into "3", which means a result of the medical test action MT3 is abnormal. The data bit DT4 in the state ST4 of the medical test action MT4 is changed into "2", which means a result of the medical test action MT4 is abnormal. After the results of the medical test actions are collected into the state ST4. Operation S244 is performed to switch the control method 200 into the result prediction stage eDIS.

Each data bit DT1-DT5 of the medical test data bits DT can be configured to -1 (means the medical test result is normal) or other number such as 1, 2 or 3 (means the medical test result is abnormal, over standard or below standard) or 0 (an unconfirmed status means it is not sure whether the medical test result is normal or abnormal). For example, in some embodiments, the data bit DT3 changed into "3" may indicate the result the medical test action MT3 is over the standard range. In some embodiments, the data bit DT4 changed into "2" may indicate the result the medical test action MT3 is below the standard range. The data bit "2" or "3" indicates different types of abnormality.

As shown in FIG. 5D, the updated state ST4 (i.e., the input state ST4 into the next round), has only include information about three symptoms and three medical tests. It is hard to tell a whole picture of the symptoms and results of all medical tests on the patient, because most of the symptoms remains unconfirmed and most results of medical tests are not available. In the embodiments, a possibility distribution of symptom features (including possibilities of unconfirmed symptom DS1, DS2, DS4, DS5, DS7 and DS9) and a possibility distribution of results of medical tests (including possibilities of unconfirmed medical tests MT2 and MT5) are calculated according to the fourth result state RST4.

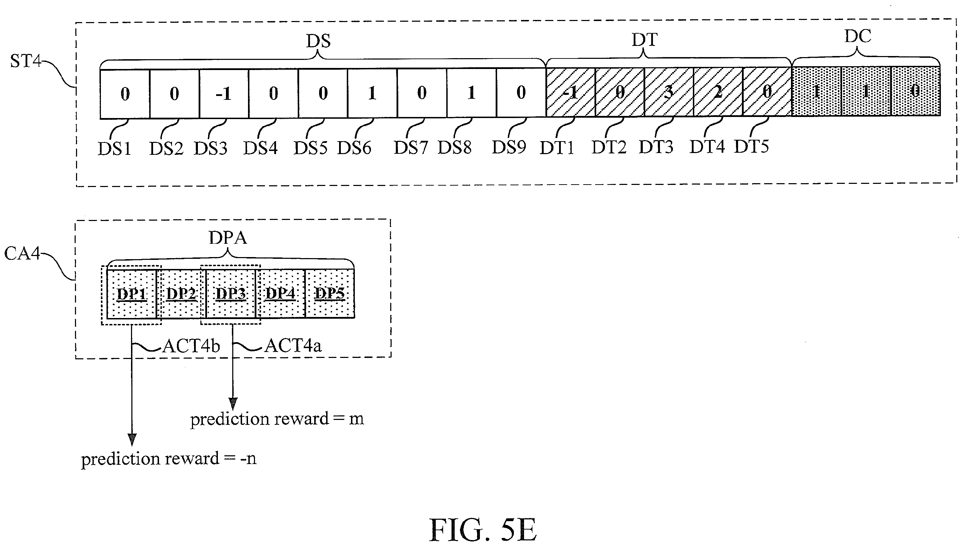

Reference is further made to FIG. 5E, which is a schematic diagram illustrating states ST4 and action ACT4a/ACT4b determined by the control method 200 in the result prediction stage eDIS in some embodiments.

As shown in FIG. 1, FIG. 2B and FIG. 5E, operation S245 is performed to determine the input state (the states ST4). The input state includes the initial state (e.g., DS6, and DC1-DC3), the previous symptom inquiry answers (e.g., DS3 and DS8) and results (e.g., DT1, DT3 and DT4) of the medical test actions (e.g., MT1, MT3 and MT4) selected in the operation S237.

Operation S246 is performed to determine, by the reinforcement learning agent 140 with the neural network model NNM, to determine priority values (e.g., Q values) of all candidate actions CA4 (which include five result prediction actions DP1-DP5 corresponding to five different diseases) in the result prediction stage eDIS according to the state ST4. In the embodiments shown in FIG. 5E, the reinforcement learning agent 140 with the neural network model NNM determines Q values of the result prediction actions DP1-DP5, according to the third result state RST3 generated by the third branch neural network portion B3 corresponding to the state ST4. In this embodiments, the third result state RST3 is generated according to answers of symptom inquiries (e.g., the patient has chest pain, difficulty to sleep but does not lose his/her appetite) and also the results of medical tests (e.g., the result of chest x-ray is abnormal, the result of otolaryngology examination is abnormal, and the result of bacterial culture test is normal).

In this case, that the third result state RST3 will have higher accuracy to reflect the priority values (Q values) of the result prediction actions DP1-DP5 because the results of medical tests may provide important and critical information for diagnosing diseases.

In the embodiment, it is assumed that the medical record MR1 in the training data TD indicates the patient has the disease corresponding to the result prediction action DP3. If the control method 200 selects the result prediction action DP3 as a current act ACT4a in operation S246, the control method 200 will give a positive prediction reward the reinforcement learning agent 140 with the neural network model NNM for making the correct prediction. On the other hand, if the control method 200 selects any other result prediction action (e.g., select the result prediction action DP1 as a current act ACT4b) in operation S246, the control method 200 will give a negative prediction reward to the reinforcement learning agent 140 with the neural network model NNM for making a wrong prediction.

In some embodiments, the control method 200 will provides a label-guided exploration probability ε. The label-guided exploration probability ε is a percentage from 0% to 100%. In some embodiments, the label-guided exploration probability ε can be in a range between 0% and 1%. In some embodiments, the label-guided exploration probability ε can be 0.5%. The label-guided exploration probability ε is utilized to speed up the training of the neural network model NNM.

In response to that a random value between 0 and 1 matches the label-guided exploration probability ε, the control method 200 provide the correct answer (the diagnosed disease in the medical records MR1) to the neural network model NNM as the result prediction action, so as to guide the neural network model NNM. In other words, there is a 0.5% chance (if ε = 0.5%), the control method 200 will direct give the correct answer of the result prediction action, such that the neural network model NNM will learn the correct answer in this case.

On the other hand, when the random value fails to match the label-guided exploration probability, the neural network model NNM is utilized to select the result prediction action. In other words, in most cases (99.5%, if ε = 0.5%), the neural network model NNM will make the prediction, and learns from the reward corresponding to the correctness of the prediction.

When the operation S230 is finished, the neural network model NNM has been utilized to select the symptom inquiry actions, the medical test actions and the result prediction action. The control method 200 goes to operation S250 for giving cumulative rewards to the reinforcement learning agent 140 with the neural network model NNM in response to aforesaid actions.

In this case, when the random value between 0 and 1 matches the label-guided exploration probability ε, the neural network model NNM will be trained according to the correct labelled data (directly from the training data TD). It is more efficient for the neural network model NNM to learn the correct labelled data contrast to randomly predicting a label and learning a failed outcome. Therefore, the label-guided exploration probability ε is utilized to speed up the training of the neural network model NNM.

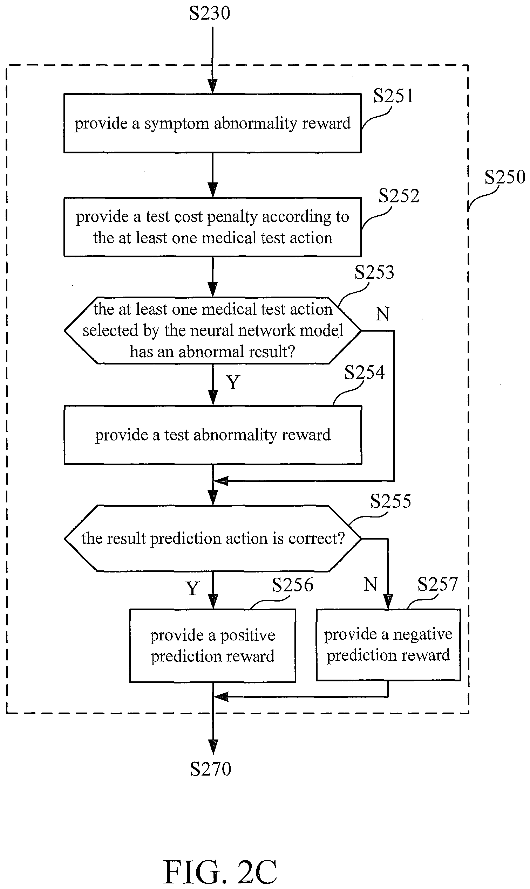

Reference is further made to FIG. 2C, which is a flow chart illustrating further operations S251-S257 in operation S250 shown in FIG. 2A according to some embodiments.

As shown in FIG. 1, FIG. 2C and FIG. 5D, operation S251 is performed by the interaction system 120 to provide a symptom abnormality reward according to the symptom inquiry answers of the symptom inquiry actions. As the embodiments shown in FIG. 5D, the input state ST4 include the data bits DS6 and DS8 labelled as "1", and it means that the patient has these two symptoms S6 and S8. The symptom abnormality reward is generated according to an amount of the symptoms, which are asked and confirmed on the patient. It is assumed that when one symptom inquiry action has the abnormal result (i.e., the patient has the symptom), one unit of symptom abnormality reward "σ" will be provided. As shown in FIG. 5D, there are two symptoms with the abnormal results, so the symptom abnormality reward will be $\sigma*2$ correspondingly.

As shown in FIG. 1, FIG. 2C and FIG. 5D, operation S252 is performed by the interaction system 120 to provide a test cost penalty according to at least one medical test selected in the combination (referring to operation S242 in FIG. 2B) to the reinforcement learning agent 140 with the neural network model NNM. In the embodiment shown in FIG. 5D, the medical tests MT1, MT3 and MT4 are selected. Therefore, the test cost penalty is decided according to a sum of costs (C1+C3+C4) of the medical tests MT1, MT3 and MT4. The test cost penalty is utilized to constrain a total amount of the medical tests suggested by the reinforcement learning agent 140 with the neural network model NNM. If there is no penalty while selecting more medical tests, the neural network model NNM will tend to select as many medical tests (which may include some unnecessary medical tests) as possible to gain the maximal rewards.

In some embodiments, the cost C1 of the medical test MT1 is decided according to a price for performing the medical test MT1, a time for performing the medical test MT1, a difficulty or risk for performing the medical test MT1, a level of unconformable of the patient under the medical test MT1. Similar, the costs C3 and C4 are decided individually about the medical test MT3 and MT4.

In some other embodiments, the costs C1, C3 and C4 can also be an approximate value equally.

When more medical tests are selected into the combination in operation S242 in FIG. 2B, the test cost penalty will be higher.

As shown in FIG. 1, FIG. 2C and FIG. 5D, operation S253 is performed to determine whether the medical test actions selected in the combination (referring to operation S242 in FIG. 2B) have abnormal results. In the embodiment shown in FIG. 5D, the medical test actions MT3 and MT4 have abnormal results and the medical test action MT1 has the normal result. Operation S254 is performed by the interaction system 120 to provide test abnormality rewards corresponding to the medical test actions MT3 and MT4 with the abnormal results. The test abnormality rewards are provided to the reinforcement learning agent 140 with the neural network model NNM. It is assumed that when one medical test action has the abnormal result, the test abnormality reward "λ" will be provided. As shown in FIG. 5D, there are two medical test actions MT3 and MT4 with the abnormal results, such that the test abnormality reward will be $\lambda*2$ corresponding to the medical test actions MT3 and MT4. The symptom abnormality rewards and the test abnormality rewards can encourage the neural network model NNM to select critical symptom inquiries or critical medical tests. In most cases, the symptoms occur on the patient will provide more information for diagnosing, compared to an answer about a symptom not occurring on the patient. In most cases, the medical tests with abnormal results will provide more information for diagnosing, compared to the medical tests with normal results.

As shown in FIG. 1, FIG. 2C and FIG. 5E, operation S255 is performed to determine whether the selected result prediction actions (referring to operation S246 in FIG. 2B) is correct or not.

As shown in FIG. 5E, if the result prediction action DP3 is selected, operation S256 is performed by the interaction system 120 to provide the positive prediction reward, +m, to the reinforcement learning agent 140. In this case, the cumulative rewards collected by the reinforcement learning agent will be:

$$m + (\sigma*2) + (\lambda*2) - (C1+C3+C4)$$

As shown in FIG. 5E, if the result prediction action DP1 is selected, operation S257 is performed by the interaction system 120 to provide the negative prediction reward, -n, to the reinforcement learning agent 140. In this case, the cumulative rewards collected by the reinforcement learning agent will be:

$$(-n) + (\sigma*2) + (\lambda*2) - (C1+C3+C4)$$

Afterward, as shown in FIG. 2A, the operation S270 is performed by the reinforcement learning agent 140 to train the neural network model NNM in reference with the cumulative rewards, which include the test abnormality reward, the prediction reward and the test cost penalty above. It is to be noticed that, the neural network model NNM is trained to maximize the cumulative rewards collected by the reinforcement learning agent 140.

Therefore, the neural network model NNM is trained to make the correct disease prediction to get the positive prediction reward. In the meantime, the neural network model NNM is trained to select the suitable combination of medical test actions, which may detect as many abnormal results as possible, and avoid selecting too many medical tests for controlling the test cost penalty.

In addition, the neural network model NNM is also trained to ask proper symptom inquiry (in order to predict the correct disease prediction to obtain the positive prediction rewards).

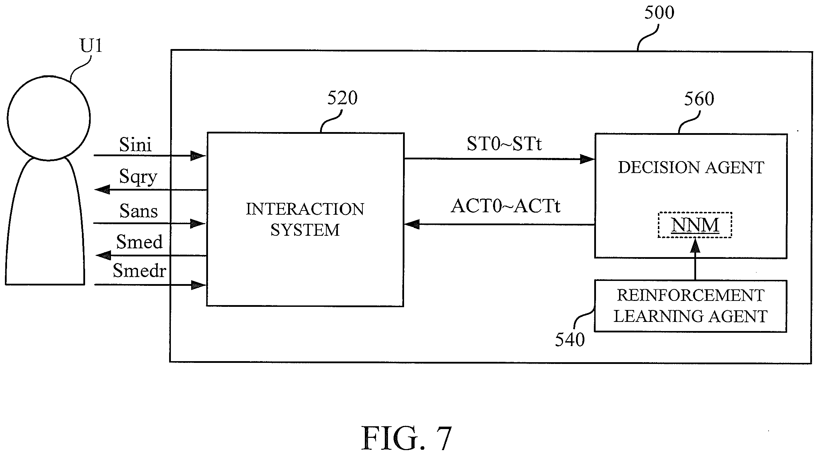

After the neural network model NNM is trained according to the control method 200 in FIG. 2A to FIG. 2C, the medical system 100 in FIG. 1 is able to be utilized to interact with a patient and provide a disease prediction to the patient according to an initial symptom and patient's answers to the symptom inquiries. Reference is made to FIG. 7, which is a schematic diagram illustrating the medical system 500 after the training of the neural network model NNM is done. In this case, the interaction system 520 may include an input/output interface, such as keyboard, mouse, microphone, touch panel or any equivalent device, to interact with a user U1. As shown in FIG. 7, the medical system 500 further include a decision agent 560, which utilize the neural network model NNM trained by the reinforcement learning agent 540.

The medical system 500 is configured to interact with the user U1 through the input/output interface (e.g. collecting an initial symptom from the user U1, providing some symptom inquiries to the user U1, collecting corresponding symptom responses from the user U1, suggesting one or more medical tests to the users and collecting results of the medical tests). Based on aforesaid interaction history, the medical system 500 is able to analyze, suggest some medical tests, diagnose or predict a potential disease occurring to the user U1.

In some embodiments, the medical system 500 is established with a computer, a server or a processing center. The interaction system 520, the reinforcement learning agent 540 and the decision agent 560 can be implemented by a processor, a central processing unit or a computation unit. In some embodiments, the interaction system 520 can further include an output interface (e.g., a display panel for display information) and an input device (e.g., a touch panel, a keyboard, a microphone, a scanner or a flash memory reader) for user to type text commands, to give voice commands or to upload some related data (e.g., images, medical records, or personal examination reports).

In some other embodiments, at least a part of the medical system 500 is established with a distribution system. For example, the interaction system 520, the reinforcement learning agent 540 and the decision agent 560 can be established by a cloud computing system.

As shown in FIG. 7, the input/output interface of the interaction system 520 can be manipulated by a user U1. The user U1 can see the information displayed on the input/output interface and the user U1 can enter his/her inputs on the input/output interface. In an embodiment, the input/output interface will display a notification to ask the user U1 about his/her symptoms. The first symptom inputted by the user U1 will be regarded as an initial symptom Sini. The input/output interface is configured for collecting the initial symptom Sini according to the user's manipulation as the state ST0. The interaction system 520 transmits the state ST0 to the decision agent 560.

The decision agent 560 is configured for selecting sequential actions ACT0-ACTt. The sequential actions ACT0-ACTt include symptom inquiry actions, medical test actions, and a result prediction action. The result prediction action can be a disease predication action and/or a medical department recommendation action corresponding to the disease prediction action. The interaction system 520 will generate symptom inquiries Sqry, medical test actions Smed according to the sequential actions ACT0-ACTt. The symptom inquiries Sqry are displayed sequentially, and the user U1 can answer the symptom inquiries Sqry. The interaction system 520 is configured for receiving symptom responses Sans corresponding to the symptom inquiries Sqry, receiving results Smedr of the medical test actions Smed. The interaction system 520 converts the symptom responses Sans and the results Smedr into the states ST1-STt. After a few inquiries (when the budget is expired), the medical system 500 shown in FIG. 7 will provide a disease prediction or a medical department recommendation to the user according to the result prediction action.

The decision agent 560 will decide optimal questions (i.e., the symptom inquiries Sqry) to ask the user U1 according to the initial symptom Sini and all previous responses Sans (before the current question), and also an optimal suggestion of medical tests based on the trained neural network model NNM.

## Claims

1. A medical system, comprising:

   an interaction system, configured for receiving an initial symptom;
   a reinforcement learning agent interacting with the interaction system,
   a decision agent interacting with the interaction system; and
   a neural network model, utilized by the decision agent to select symptom inquiry actions according to the initial symptom;
   wherein the neural network model is trained by the reinforcement learning agent according to training data, the training data comprising a plurality of medical records, each one of the medical records comprising a diagnosed disease, a plurality of first medical test results performed for diagnosing the diagnosed disease, and a plurality of diagnosed symptoms related to the diagnosed disease,
   wherein the neural network model is utilized by the reinforcement learning agent for selecting at least one first medical test action from first candidate actions and to select a first disease prediction action from second candidate actions according to the training data,
   wherein the interaction system determines a first input state comprising symptom inquiry answers of the symptom inquiry actions, wherein the symptom inquiry answers are determined according to the diagnosed symptoms in the medical record of the training data,
   wherein the interaction system provides a test cost penalty to the reinforcement learning agent according to the at least one first medical test action,
   wherein the interaction system provides a test abnormality reward to the reinforcement learning agent according to the plurality of first medical test results in the training data corresponding to the at least one first medical test action,
   wherein the interaction system provides a prediction reward to the reinforcement learning agent according to a comparison between the first disease prediction action and the diagnosed disease in the medical records, and
   wherein the neural network model is trained to maximize cumulative rewards in reference with the test abnormality reward, the prediction reward, and the test cost penalty;
   wherein the interaction system is configured to receive symptom inquiry answers in response to the symptom inquiry actions,
   wherein the neural network model is utilized by the decision agent to select at least one second medical test action from the first candidate actions according to the initial symptom and the at least one symptom answer,
   wherein the interaction system is configured to receive at least one second test result of the at least one second medical test action,
   wherein the neural network model is utilized by the decision agent to select a second disease prediction action and/or a medical department recommendation for the second disease prediction action from the second candidate actions according to the initial symptom, the symptom inquiry answers, and the at least one second test result;
   wherein the neural network model comprises a common neural network portion, a first branch neural network portion, a second branch neural network portion and a third branch neural network portion,
   wherein the first branch neural network portion and the second branch neural network portion and the third branch neural network portion are respectively connected to the common neural network portion, and
   wherein a first result state generated by the first branch neural network portion is utilized to select the symptom inquiry actions, a second result state generated by the second branch neural network portion is utilized to select the at least one first and second medical test action, and a third result state generated by the third branch neural network portion is utilized to select the first and second disease prediction action and/or a medical department recommendation for the first and second disease prediction action.

2. The medical system as claimed in claim 1,

   wherein the interaction system determines a second input state comprising the initial symptom, the symptom inquiry answers and the at least one second medical test result corresponding to the at least one second medical test action, and

wherein the reinforcement learning agent selects the second disease prediction action and/or a medical department recommendation for the second disease prediction action according to the second input state.

3. The medical system as claimed in claim 1, wherein a combination of medical test actions are selected from the first candidate actions simultaneously according to the first input state, and the reinforcement learning agent generates probability values of the first candidate actions and complement probability values of the first candidate actions by the neural network model according to the first input state,

wherein the reinforcement learning agent determines a plurality of weights of all combinations of the first candidate actions according to the probability values and the complement probability values, and
wherein the reinforcement learning agent selects the combination of medical test actions from the all combinations of the first candidate actions in reference with the weights.

4. The medical system as claimed in claim 1, wherein the first branch neural network portion and the third branch neural network portion adopt first activation functions, and the second branch neural network portion adopts a second activation function different from the first activation functions.

**Patentansprüche**

1. Ein medizinisches System, bestehend aus:

ein Interaktionssystem, das für den Empfang eines Anfangssymptoms konfiguriert ist;
einen Verstärkungslernagenten, der mit dem Interaktionssystem interagiert,
einen Entscheidungsagenten, der mit dem Interaktionssystem interagiert; und
ein neuronales Netzmodell, das von dem Entscheidungsagenten verwendet wird, um Maßnahmen zur Symptomermittlung in Abhängigkeit von dem Ausgangssymptom auszuwählen;
wobei das neuronale Netzmodell durch den Verstärkungslernagenten gemäß Trainingsdaten trainiert wird, wobei die Trainingsdaten eine Vielzahl von medizinischen Aufzeichnungen umfassen, wobei jede der medizinischen Aufzeichnungen eine diagnostizierte Krankheit, eine Vielzahl von ersten medizinischen Testergebnissen, die zur Diagnose der diagnostizierten Krankheit durchgeführt wurden, und eine Vielzahl von diagnostizierten Symptomen, die mit der diagnostizierten Krankheit zusammenhängen, umfasst,
wobei das neuronale Netzmodell von dem Verstärkungslernagenten verwendet wird, um mindestens eine erste medizinische Testaktion aus ersten Kandidatenaktionen auszuwählen und um eine erste Krankheitsvorhersageaktion aus zweiten Kandidatenaktionen gemäß den Trainingsdaten auszuwählen,
wobei das Interaktionssystem einen ersten Eingabezustand bestimmt, der Symptomabfrageantworten der Symptomabfrageaktionen umfasst, wobei die Symptomabfrageantworten gemäß den diagnostizierten Symptomen in der Krankenakte der Trainingsdaten bestimmt werden,
wobei das Interaktionssystem dem Verstärkungslernagenten eine Testkostenstrafe in Abhängigkeit von der mindestens einen ersten medizinischen Testaktion zuweist,
wobei das Interaktionssystem eine Testabnormalitätsbelohnung an den Verstärkungslernagenten entsprechend der Vielzahl von ersten medizinischen Testergebnissen in den Trainingsdaten bereitstellt, die der mindestens einen ersten medizinischen Testaktion entsprechen,
wobei das Interaktionssystem dem Verstärkungslernagenten eine Vorhersagebelohnung entsprechend einem Vergleich zwischen der ersten Krankheitsprognoseaktion und der diagnostizierten Krankheit in den medizinischen Aufzeichnungen bereitstellt, und
wobei das neuronale Netzmodell so trainiert wird, dass es die kumulativen Belohnungen in Bezug auf die Testabnormalitätsbelohnung, die Vorhersagebelohnung und die Testkostenstrafe maximiert;
wobei das Interaktionssystem so konfiguriert ist, dass es Antworten auf Symptomabfragen als Reaktion auf die Symptomabfrageaktionen empfängt,
wobei das neuronale Netzmodell von dem Entscheidungsagenten verwendet wird, um mindestens eine zweite medizinische Testaktion aus den ersten Aktionskandidaten in Abhängigkeit von dem Anfangssymptom und der mindestens einen Symptomantwort auszuwählen,
wobei das Interaktionssystem so konfiguriert ist, dass es mindestens ein zweites Testergebnis der mindestens einen zweiten medizinischen Testaktion empfängt,
wobei das neuronale Netzwerkmodell von dem Entscheidungsagenten verwendet wird, um eine zweite Krankheitsprognosemaßnahme und/oder eine Empfehlung der medizinischen Abteilung für die zweite Krankheitsprognosemaßnahme aus den zweiten Kandidatenmaßnahmen entsprechend dem Anfangssymptom, den Antworten auf die Symptomabfrage und dem mindestens einen zweiten Testergebnis auszuwählen;

wobei das neuronale Netzwerkmodell einen gemeinsamen neuronalen Netzwerkteil, einen ersten Zweig eines neuronalen Netzwerks, einen zweiten Zweig eines neuronalen Netzwerks und einen dritten Zweig eines neuronalen Netzwerks umfasst,

wobei der erste Zweig des neuronalen Netzes und der zweite Zweig des neuronalen Netzes und der dritte Zweig des neuronalen Netzes jeweils mit dem gemeinsamen neuronalen Netz verbunden sind, und

wobei ein erster Ergebniszustand, der durch den ersten Zweig des neuronalen Netzes erzeugt wird, verwendet wird, um die Symptomerhebungsaktionen auszuwählen, ein zweiter Ergebniszustand, der durch den zweiten Zweig des neuronalen Netzes erzeugt wird, verwendet wird, um die mindestens eine erste und zweite medizinische Testaktion auszuwählen, und ein dritter Ergebniszustand, der durch den dritten Zweig des neuronalen Netzes erzeugt wird, verwendet wird, um die erste und zweite Krankheitsprognoseaktion und/oder eine Empfehlung der medizinischen Abteilung für die erste und zweite Krankheitsprognoseaktion auszuwählen.

2. Das medizinische System nach Anspruch 1,

wobei das Interaktionssystem einen zweiten Eingabezustand bestimmt, der das Anfangssymptom, die Antworten auf die Symptomabfrage und das mindestens eine zweite medizinische Testergebnis umfasst, das der mindestens einen zweiten medizinischen Testaktion entspricht, und

wobei der Verstärkungslernagent die zweite Krankheitsvorhersageaktion und/oder eine Empfehlung der medizinischen Abteilung für die zweite Krankheitsvorhersageaktion entsprechend dem zweiten Eingabezustand auswählt.

3. Medizinisches System nach Anspruch 1, wobei eine Kombination von medizinischen Testaktionen aus den ersten Kandidatenaktionen gleichzeitig gemäß dem ersten Eingabezustand ausgewählt wird und der Verstärkungslernagent Wahrscheinlichkeitswerte der ersten Kandidatenaktionen und Komplement-Wahrscheinlichkeitswerte der ersten Kandidatenaktionen durch das neuronale Netzwerkmodell gemäß dem ersten Eingabezustand erzeugt,

wobei der Verstärkungslernagent eine Vielzahl von Gewichten aller Kombinationen der ersten Aktionskandidaten gemäß den Wahrscheinlichkeitswerten und den Komplement-Wahrscheinlichkeitswerten bestimmt, und

wobei der Verstärkungslernagent die Kombination von medizinischen Testaktionen aus allen Kombinationen der ersten Kandidatenaktionen in Bezug auf die Gewichte auswählt.

4. Medizinisches System nach Anspruch 1, wobei der erste Zweig des neuronalen Netzes und der dritte Zweig des neuronalen Netzes erste Aktivierungsfunktionen annehmen, und der zweite Zweig des neuronalen Netzes eine zweite Aktivierungsfunktion annimmt, die sich von den ersten Aktivierungsfunktionen unterscheidet.

**Revendications**

1. Un système médical comprenant:

un système d'interaction, configuré pour recevoir un symptôme initial;

un agent d'apprentissage par renforcement interagissant avec le système d'interaction,

un agent de décision interagissant avec le système d'interaction; et

un modèle de réseau neuronal, utilisé par l'agent de décision pour sélectionner les actions de recherche de symptômes en fonction du symptôme initial;

Le modèle de réseau neuronal est entraîné par l'agent d'apprentissage par renforcement en fonction de données d'entraînement, les données d'entraînement comprenant une pluralité de dossiers médicaux, chacun des dossiers médicaux comprenant une maladie diagnostiquée, une pluralité de premiers résultats de tests médicaux effectués pour diagnostiquer la maladie diagnostiquée, et une pluralité de symptômes diagnostiqués liés à la maladie diagnostiquée,

Le modèle de réseau neuronal est utilisé par l'agent d'apprentissage par renforcement pour sélectionner au moins une première action de test médical parmi les premières actions candidates et pour sélectionner une première action de prédiction de la maladie parmi les secondes actions candidates en fonction des données d'apprentissage,

le système d'interaction détermine un premier état d'entrée comprenant les réponses aux demandes de renseignements sur les symptômes des actions de demande de renseignements sur les symptômes, les réponses aux demandes de renseignements sur les symptômes étant déterminées en fonction des symptômes diagnostiqués dans le dossier médical des données d'apprentissage,

dans lequel le système d'interaction fournit une pénalité de coût de test à l'agent d'apprentissage par renforcement en fonction d'au moins une première action de test médical,

Le système d'interaction fournit une récompense pour anomalie de test à l'agent d'apprentissage par renforcement en fonction de la pluralité de résultats du premier test médical dans les données d'apprentissage correspondant à l'au moins une action du premier test médical,

le système d'interaction fournit une récompense de prédiction à l'agent d'apprentissage par renforcement en fonction d'une comparaison entre la première action de prédiction de la maladie et la maladie diagnostiquée dans le dossier médical, et

Le modèle de réseau neuronal est entraîné pour maximiser les récompenses cumulées en référence à la récompense de l'anomalie du test, à la récompense de la prédiction et à la pénalité du coût du test;

le système d'interaction est configuré pour recevoir des réponses à la demande de renseignements sur les symptômes en réponse aux actions de demande de renseignements sur les symptômes,

le modèle de réseau neuronal est utilisé par l'agent de décision pour sélectionner au moins une deuxième action de test médical parmi les premières actions candidates en fonction du symptôme initial et d'au moins une réponse au symptôme,

le système d'interaction est configuré pour recevoir au moins un deuxième résultat de test de l'au moins une deuxième action de test médical,

le modèle de réseau neuronal est utilisé par l'agent de décision pour sélectionner une deuxième action de prédiction de la maladie et/ou une recommandation du service médical pour la deuxième action de prédiction de la maladie parmi les deuxièmes actions candidates en fonction du symptôme initial, des réponses à la demande de renseignements sur les symptômes et d'au moins un deuxième résultat de test;

le modèle de réseau neuronal comprend une partie de réseau neuronal commun, une partie de réseau neuronal de première branche, une partie de réseau neuronal de deuxième branche et une partie de réseau neuronal de troisième branche,

dans lequel la première branche du réseau neuronal, la deuxième branche du réseau neuronal et la troisième branche du réseau neuronal sont respectivement connectées à la partie commune du réseau neuronal, et

dans lequel un premier état de résultat généré par la première partie du réseau neuronal de branche est utilisé pour sélectionner les actions de recherche de symptômes, un deuxième état de résultat généré par la deuxième partie du réseau neuronal de branche est utilisé pour sélectionner au moins une première et une deuxième action de test médical, et un troisième état de résultat généré par la troisième partie du réseau neuronal de branche est utilisé pour sélectionner la première et la deuxième action de prédiction de maladie et/ou une recommandation du service médical pour la première et la deuxième action de prédiction de maladie.

2. Système médical selon la revendication 1,

dans lequel le système d'interaction détermine un deuxième état d'entrée comprenant le symptôme initial, les réponses à la demande de renseignements sur les symptômes et au moins un deuxième résultat de test médical correspondant à au moins une deuxième action de test médical, et

l'agent d'apprentissage par renforcement sélectionne la deuxième action de prédiction de la maladie et/ou une recommandation du service médical pour la deuxième action de prédiction de la maladie en fonction du deuxième état d'entrée.

3. Système médical selon la revendication 1, dans lequel une combinaison d'actions de test médical est sélectionnée simultanément parmi les premières actions candidates en fonction du premier état d'entrée, et l'agent d'apprentissage par renforcement génère des valeurs de probabilité des premières actions candidates et complète les valeurs de probabilité des premières actions candidates par le modèle de réseau neuronal en fonction du premier état d'entrée,

l'agent d'apprentissage par renforcement détermine une pluralité de poids pour toutes les combinaisons des premières actions candidates en fonction des valeurs de probabilité et des valeurs de probabilité complémentaires, et

L'agent d'apprentissage par renforcement sélectionne la combinaison d'actions de test médical parmi toutes les combinaisons des premières actions candidates en fonction des poids.

4. Système médical selon la revendication 1, dans lequel la première branche du réseau neuronal et la troisième branche du réseau neuronal adoptent des premières fonctions d'activation, et la deuxième branche du réseau neuronal adopte une deuxième fonction d'activation différente des premières fonctions d'activation.

FIG. 1

EP 3 618 080 B1

S210                    200

```
┌─────────────────────────────────────┐
│          obtain training data        │
└─────────────────────────────────────┘
                    │
                    ▼
┌─────────────────────────────────────┐
│   utilize the neural network model to │  S230
│ select symptom inquiry actions, at least│
│   one medical test action and a result │
│            prediction action          │
└─────────────────────────────────────┘
                    │
                    ▼
┌─────────────────────────────────────┐
│  provide cumulative rewards in response│  S250
│ to actions selected by the neural network│
│                 model                 │
└─────────────────────────────────────┘
                    │
                    ▼
┌─────────────────────────────────────┐  S270
│     train the neural network model to │
│      maximize cumulative rewards      │
└─────────────────────────────────────┘
```

# FIG. 2A

S210

S230

determine a current stage? S231

current stage = eDIS

current stage = eSYM

S232

current stage = eMED

S239

| determine an input state including an initial state and previous symptom inquiry answers |
|---|

| determine an input state including an initial state and previous symptom inquiry answers |
|---|

S233

| determine priority values of SQ1-SQ9, action for switching into eMED, and action for switching into eDIS according to the input state |
|---|

S240

| determine probability values and complement probability values of MT1-MT5 according to the input state |
|---|

S241

| determine weights of all combinations of MT1-MT5 |
|---|

S234

Q1

search for the highest priority value

Q2

SQ1-SQ9

S242

| select one combination of medical test actions |
|---|

| switch into eMED |
|---|
S235

| select a symptom inquiry action |
|---|
S237

S243

| collect medical test results |
|---|

| switch into eDIS |
|---|
S236

| collect a symptom inquiry answer |
|---|
S238

S244

| switch into eDIS |
|---|

S245

| determine an input state including an initial state, previous symptom inquiry answers and medical test results in the selected combination |
|---|

S246

| select a result prediction action according to the input state |
|---|

FIG. 2B

S250

S230

↓

S251
provide a symptom abnormality reward

↓

S252
provide a test cost penalty according to
the at least one medical test action

↓

S253
the at least one medical test action
selected by the neural network model
has an abnormal result?

N →

Y ↓

S254
provide a test abnormality reward

↓

S255
the result prediction action is correct?

Y ↓ S256

N ↓ S257

provide a positive
prediction reward

provide a negative
prediction reward

S250

↓

S270

# FIG. 2C

MR1

TDS | TDT | TDC

| 1 | 0 | 0 | 0 | 0 | 1 | 0 | 1 | 0 | -1 | -1 | 3 | 2 | -1 | 1 | 1 | 0 |
|---|---|---|---|---|---|---|---|---|----|----|---|---|----|---|---|---|

S1  S2  S3  S4  S5  S6  S7  S8  S9  MT1 MT2 MT3  MT4 MT5

FIG. 3

FIG. 4

DS | DT | DC

ST0

| DS1 | DS2 | DS3 | DS4 | DS5 | DS6 | DS7 | DS8 | DS9 | DT1 | DT2 | DT3 | DT4 | DT5 | DC1 | DC2 | DC3 |
| 0 | 0 | 0 | 0 | 0 | 1 | 0 | 0 | 0 | 0 | 0 | 0 | 0 | 0 | 1 | 1 | 0 |

SQA

CA0

| SQ1 | SQ2 | SQ3 | SQ4 | SQ5 | SQ6 | SQ7 | SQ8 | SQ9 | Q1 | Q2 |

$\rightarrow$ eDIS

$\rightarrow$ eMED

ACT0

DS | DT | DC

ST1

| DS1 | DS2 | DS3 | DS4 | DS5 | DS6 | DS7 | DS8 | DS9 | | | | | | | | |
| 0 | 0 | -1 | 0 | 0 | 1 | 0 | 0 | 0 | 0 | 0 | 0 | 0 | 0 | 1 | 1 | 0 |

# FIG. 5A

EP 3 618 080 B1

ST1

| DS | | | | | | | | | DT | | | | | DC | | |
|---|---|---|---|---|---|---|---|---|---|---|---|---|---|---|---|---|
| 0 | 0 | -1 | 0 | 0 | 1 | 0 | 0 | 0 | 0 | 0 | 0 | 0 | 0 | 1 | 1 | 0 |

DS1 DS2 DS3 DS4 DS5 DS6 DS7 DS8 DS9 DT1 DT2 DT3 DT4 DT5 DC1 DC2 DC3

CA1

| SQA | | | | | | | | | | |
|---|---|---|---|---|---|---|---|---|---|---|
| SQ1 | SQ2 | SQ3 | SQ4 | SQ5 | SQ6 | SQ7 | SQ8 | SQ9 | Q1 | Q2 |

→ eDIS

····▶ eMED

ACT1

ST2

| DS | | | | | | | | | DT | | | | | DC | | |
|---|---|---|---|---|---|---|---|---|---|---|---|---|---|---|---|---|
| 0 | 0 | -1 | 0 | 0 | 1 | 0 | 1 | 0 | 0 | 0 | 0 | 0 | 0 | 1 | 1 | 0 |

DS1 DS2 DS3 DS4 DS5 DS6 DS7 DS8 DS9

FIG. 5B

ST2

DS | DT | DC

| DS1 | DS2 | DS3 | DS4 | DS5 | DS6 | DS7 | DS8 | DS9 | DT1 | DT2 | DT3 | DT4 | DT5 | DC1 | DC2 | DC3 |
|---|---|---|---|---|---|---|---|---|---|---|---|---|---|---|---|---|
| 0 | 0 | -1 | 0 | 0 | 1 | 0 | 1 | 0 | 0 | 0 | 0 | 0 | 0 | 1 | 1 | 0 |

CA2

SQA | ACT2

| SQ1 | SQ2 | SQ3 | SQ4 | SQ5 | SQ6 | SQ7 | SQ8 | SQ9 | Q1 | Q2 |
|---|---|---|---|---|---|---|---|---|---|---|

→eDIS

→eMED

ST3

DS | DT | DC

| DS1 | DS2 | DS3 | DS4 | DS5 | DS6 | DS7 | DS8 | DS9 | | | | | | | | |
|---|---|---|---|---|---|---|---|---|---|---|---|---|---|---|---|---|
| 0 | 0 | -1 | 0 | 0 | 1 | 0 | 1 | 0 | 0 | 0 | 0 | 0 | 0 | 1 | 1 | 0 |

FIG. 5C

FIG. 5D

EP 3 618 080 B1

ST4

DS

| 0 | 0 | -1 | 0 | 0 | 1 | 0 | 1 | 0 |

DT

| -1 | 0 | 3 | 2 | 0 |

DC

| 1 | 1 | 0 |

DS1  DS2  DS3  DS4  DS5 DS6  DS7 DS8  DS9 DT1  DT2  DT3  DT4 DT5

CA4

DPA

| DP1 | DP2 | DP3 | DP4 | DP5 |

ACT4b    ACT4a

prediction reward = m

prediction reward = -n

FIG. 5E

EP 3 618 080 B1

EP 3 618 080 B1

|  | MT1 | MT2 | MT3 | MT4 | MT5 |
|---|---|---|---|---|---|
| probability value | 0.4 | 0.2 | 0.7 | 1 | 0 |
| complement probability value | 0.6 | 0.8 | 0.3 | 0 | 1 |

## FIG. 6A

| CMB1 | CMB2 | CMB3 | CMB4 | CMB5 | CMB6 | CMB7 | CMB8 |
|---|---|---|---|---|---|---|---|
| MT4 | MT1+MT4 | MT2+MT4 | MT3+MT4 | MT1+MT2+MT4 | MT1+MT3+MT4 | MT2+MT3+MT4 | MT1+MT2+MT3+MT4 |
| W1 | W2 | W3 | W4 | W5 | W6 | W7 | W8 |

## FIG. 6B

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018046773 A1